Europäisches Patentamt

European Patent Office

Office européen des brevets

(11) Veröffentlichungsnummer: **0 219 608**
A2

(12) # EUROPÄISCHE PATENTANMELDUNG

(21) Anmeldenummer: 86108210.5

(22) Anmeldetag: 16.06.86

(51) Int. Cl.⁴: **A 61 N 1/05**

(30) Priorität: 26.09.85 DE 3534281

(43) Veröffentlichungstag der Anmeldung:
29.04.87 Patentblatt 87/18

(84) Benannte Vertragsstaaten:
AT CH DE FR GB IT LI NL SE

(71) Anmelder: Osypka, Peter, Dr. Ing.
Basler Strasse 109
D-7889 Grenzach-Wyhlen(DE)

(72) Erfinder: Osypka, Peter, Dr. Ing.
Basler Strasse 109
D-7889 Grenzach-Wyhlen(DE)

(74) Vertreter: Schmitt, Hans, Dipl.-Ing. et al,
Patentanwälte Dipl.-Ing H. Schmitt Dipl.-Ing. W. Maucher
Dreikönigstrasse 13
D-7800 Freiburg(DE)

(54) Transvenös einführbare Herzschrittmacherleitung.

(57) Eine implantierbare, transvenös einführbare Herzschrittmacherleitung (1) hat einen flexiblen Leiter (2), welcher an seinem im Herzen zu verankernden distalen Ende eine relativ zu ihm feststehende, in den Herzmuskel einschraubbare Schraubwendel (4) aufweist. Ferner gehört zu der Herzschrittmacherleitung (1) ein den Leiter (2) beim Implantieren umgebendes flexibles Führungsrohr (5), gegenüber welchem die Schraubwendel (4) nach dem Einführen in das Herz durch eine axiale Relativbewegung zwischen Leiter (2) und Führungsrohr (5) hervortritt und durch Drehen in den Herzmuskel einschraubbar ist. Danach wird das Führungsrohr (5) entfernt. Um dabei das proximale Ende des Leiters (2) auch während des Entfernens des Führungsrohres (5) zugänglich zu machen, damit ein Mandrin (8) eingeführt bleiben oder werden kann und vor allem ein Stecker (7) schon am Leiter (2) befestigt sein kann, hat das Führungsrohr (5) wenigstens eine über seine Länge verlaufende Sollreißstelle, an welcher es beim Zurückziehen gleichzeitig aufgetrennt werden kann, so daß es nach dem Austritt aus dem Körper seitlich von dem Leiter (2) abgezogen werden kann.

EP 0 219 608 A2

./...

Fig.3

**PATENTANWÄLTE**

DIPL.-ING. H. SCHMITT

DIPL.-ING. W. MAUCHER

78 FREIBURG I. BR.
DREIKÖNIGSTR. 13
TELEFON: (0761) 2 73
70774

0219608

Herr
Dr.-Ing. Peter Osypka
Basler Straße 109
7889 Grenzach-Wyhlen

MR/bö

**13. Juni 1986**

UNSERE AKTE · BITTE STETS ANGEBEN!

E 86 253 MR

## Transvenös einführbare Herzschrittmacherleitung

Die Erfindung betrifft eine implantierbare, transvenös einführbare Herzschrittmacherleitung mit einem flexiblen, vorzugsweise gewendelten Leiter, welcher an seinem im Herzen zu verankernden distalen Ende eine relativ zu ihm feststehende, in den Herzmuskel einschraubbare Schraubwendel aufweist, und mit einem den Leiter umgebenden flexiblen Führungsrohr, in welchem der Leiter mit seiner Isolierung während des Einführens so untergebracht ist, daß die Schraubwendel nach dem Einführen der Herzschrittmacherleitung in das Herz durch eine axiale Relativbewegung zwischen Leiter und Führungsrohr gegenüber der Mündung des Führungsrohres hervortritt und durch Drehen in den Herzmuskel einschraubbar ist, wobei nach dem Implantieren der Herzschrittmacherleitung das Führungsrohr entfernt wird.

Eine derartige Herzschrittmacherleitung ist aus dem Buch "Cardiac Pacing" (1977 Excerpta Medica), Proceedings of the Vth International Symposium Tokio 1976, Seiten 527 bis 531, insbesondere Seite 528, Abs.2, bekannt. Das Führungsrohr dient dabei dazu, während des Einführens der Herzschrittmacherleitung eine Verhakung der sehr spitzen Einschraubwendel an Krümmungen oder Unebenheiten der Vene, am Eingang

/ 2

in das Herz, an den Herzklappen usw. zu verhindern. Das anschließende Entfernen des Führungsrohres ist jedoch schwierig und macht es notwendig, daß der Stecker des Leiters, womit dieser mit dem Herzschrittmacher selbst gekuppelt werden muß, während der Implantation noch nicht angebracht sein darf. Der Stecker muß also während der Operation vom Arzt nach Entfernen des Führungsrohres zusätzlich angebracht werden. Darüber hinaus ist das Entfernen des Führungsrohres selbst schwierig, weil dieses etwa die gleiche Länge wie der Leiter hat und somit beim Herausziehen eine immer größere Verlängerung der Gesamtanordnung bewirkt, bis schließlich das distale Ende des Führungsrohres das entgegengesetzte Ende des Leiters passiert hat. Durch den beim Zurückziehen des Führungsrohres auftretenden Zug könnte außerdem der Leiter im Herzen disloziiert werden. Da das Führungsrohr eine relativ große Länge hat, ist aber ein gleichzeitiges Gegenhalten gegen diesen Zug an dem Leiter selbst kaum möglich.

Aus der DE-OS 25 33 766 ist zwar bereits eine Herzschrittmacherleitung mit Führungsrohr bekannt, bei welcher aber das Führungsrohr zugleich die bleibende isolierende Hülle des Leiters darstellt und nach der Implantation nicht entfernt wird. Dabei besteht jedoch die Notwendigkeit, daß im distalen Endbereich des Führungsrohres ein Gegengewinde für die Schraubwendel vorgesehen ist, damit diese für die Implantation und Befestigung im Herzen aus dem Führungsrohr herausgedreht werden kann. Dies führt zu einem relativ starren und dicken Elektrodenkopf, der für das chronische Reizschwellenverhalten nachteilig ist, da er eine erheblich geringere Anpassung an die Herzbewegungen ermöglicht, so daß es zu zusätzlichen Bindegewebsbildungen kommen kann.

Es besteht deshalb die Aufgabe, eine Herzschrittmacherleitung der eingangs erwähnten Art zu schaffen, bei

3    0219608

welcher die Vorteile einer Verbindung mit dem Herzen durch Einschrauben einer Schraubwendel erhalten bleiben, diese Schraubwendel ohne Verhakung mittels eines Führungsrohres in das Herz implantiert werden kann, das Führungsrohr anschließend entfernt werden kann, ohne daß die Gefahr einer Disloziierung des implantierten Leiters besteht, wobei es außerdem möglich sein soll, den Leiter im implantierten Zustand gegen die beim Entfernen des Führungsrohres aufzubringende Zugkraft festzuhalen. Außerdem soll möglich sein, daß der Leiter bereits vor der Implantation, also vorzugsweise werksseitig, mit einem Elektrodenstecker verbunden und versehen ist.

Die Lösung dieser Aufgabe besteht im wesentlichen darin, daß das Führungsrohr wenigstens eine über seine Länge verlaufende Sollreißstelle oder -linie hat.

Dadurch ist es möglich, nach dem Implantieren der Herzschrittmacherleitung das Führungsrohr entgegen der Einführrichtung nach hinten und außerhalb des Körpers seitlich von dem Leiter wegzuziehen, wobei das Führungsrohr an der insbesondere über die gesamte Länge verlaufenden Sollreißstelle entsprechend dem Fortschritt des Herausziehens geöffnet wird und somit auch das proximale Ende des Leiters während dieser Manipulation ständig zugänglich bleibt, um diesen erforderlichenfalls beispielsweise mit einem in sein Inneres passenden Mandrin od. dgl. entgegen der Zugrichtung festzuhalten.Darüber hinaus kann das Führungsrohr entfernt werden, obwohl der Stecker an dem Leiter befestigt ist.

Eine bevorzugte Ausführungsform kann darin bestehen, daß zwei vorzugsweise einander an einem Durchmesser gegenüberliegende Sollreißstellen über die Länge des Führungsrohres vorgesehen sind. Dadurch können zwei Teile oder

/ 4

Hälften des Führungsrohres erfaßt und unter gleichzeitigem Auseinandertrennen zurückgezogen werden. Dabei kann die Sollreißstelle durch eine Schwächung der Rohrwand entlang einer Meridianlinie gebildet sein.

Das Einführen der Herzschrittmacherleitung einerseits und das Zurückziehen des Führungsrohres andererseits können dadurch erleichtert sein, daß am distalen Ende der Herzschrittmacherleitung zwischen Leiter und Führungsrohr in einer Wirkrichtung Formschluß besteht, der ein Zurückziehen des Leiters in das Führungsrohr sperrt und das Zurückziehen des Führungsrohres relativ zum Leiter freigibt. Eine besonders einfache Verwirklichung dieses Formschlusses kann darin bestehen, daß der Querschnitt des Kopfes des Leiters gegenüber dem des Leiters selbst vergrößert ist und in einer entsprechenden Innendurchmesservergrößerung des distalen Endes des Führungsrohres angeordnet ist. Dabei kann die Durchmesservergrößerung des Führungsrohres durch eine Aufweitung gebildet sein.

Um das Zurückziehen des Führungsrohres mit einer oder mehreren Sollreißstellen zu erleichtern, kann an dem proximalen Ende wenigstens ein Handgriff, bei zwei Sollreißstellen vorzugsweise an jedem der voneinander zu trennenden Führungsrohr-Teile ein eigenen Handgriff vorgesehen sein. Dadurch kann das Herausziehen und Auseinandertrennen des des Führungsrohres beschleunigt und vereinfacht werden.

Es sei noch erwähnt, daß eine Sollreißstelle als vorgefertigter durchgehender Schlitz ausgebildet sein kann, der durch die Elastizität des Materiales zunächst geschlossen ist. Versuche haben gezeigt, daß aufgrund des relativ kleinen Krümmungsradius' des Querschnittes eines solchen Führungsrohres und des bevorzugt verwendeten Polyurethans als Werkstoff eine ausreichende Eigenspannung vorhanden ist, um diesen Schlitz während des Implantierens geschlossen zu halten, so daß das Führungsrohr dabei seine Aufgabe voll erfüllen kann, beim Herausziehen aber um so leichter von dem Leiter seitlich weggezogen werden kann. Dies gilt vor allem für eine Ausführungsform, bei welcher das Führungsrohr nur eine Sollreißstelle entlang einem Meridian hat.

Vor allem bei Kombination einzelner oder mehrerer der vorbeschriebenen Merkmale und Maßnahmen ergibt sich eineimplantierbare Herzschrittmacherleitung, bei welcher die Vorteile eines wieder entfernbaren Führungsrohres mit denen einer Schraubelektrode kombiniert sind, bei denen der Elektrodenstecker von vorneherein an dem Leiter befestigt sein kann, ohne die jeweiligen Nachteile in Kauf zu nehmen, daß das Herausziehen des Führungsrohres erheblich vereinfacht ist und Vorsorge dafür getroffen ist, daß das proximale Ende des Leiters auch während des Entfernens des Führungsrohres zugänglich und sichtbar bleiben kann.

Nachstehend ist die Erfindung mit ihren ihr als wesentlich zugehörenden Einzelheiten anhand der Zeichnung noch näher beschrieben.

Es zeigt in schematisierter Darstellung :

Fig. 1    eine implantierbare transvenös einführbare Herzschrittmacherleitung mit einem Führungsrohr und
einem darin angeordneten Leiter, welcher an dem
proximalen Ende mit einem Stecker versehen ist,
wobei das Führungsrohr zwei Sollreißstellen entlang seiner Erstreckung hat,

Fig. 2    die in Fig. 1 dargestellte Herzschrittmacherleitung, wobei das Führungsrohr ein erstes
Stück zurückgezogen ist,

Fig. 3   die Herzschrittmacherleitung gemäß den Fig. 1 und 2, wobei das Führungsrohr ein größeres Stück zurückgezogen und entlang seinen Sollreißlinien aufgetrennt ist, sowie

Fig. 4   eine abgewandelte Ausführungsform der Herzschrittmacherleitung, bei welcher das Führungsrohr nur eine Sollreißlinie hat und beim Zurückziehen seitlich vor dem Stecker des Leiters weggezogen wird,

Fig. 5   das Aufreißen des Führungsrohres entlang seiner und 6   Sollreißlinien beim Zurückziehen mit Hilfe eines Hilfsgerätes.

Eine im ganzen mit 1 bezeichnete Herzschrittmacherleitung, die transvenös implantierbar ist, weist einen flexiblen, vorzugsweise gewendelten Leiter 2 auf, welcher an seinem im Herzen zu verankernden distalen Ende 3 eine relativ zu ihm feststehend, in den Herzmuskel einschraubbare Schraubwendel 4 hat. Zu der Herzschrittmacherleitung 1 gehört ferner ein den Leiter 2 zunächst umgebendes flexibles Führungsrohr 5, in welchem der Leiter 2 mit seiner Isolierung und der Schraubwendel 4 während des Einführens so untergebracht ist, daß die Schraubwendel 4 nach dem Einführen in das Herz durch eine axiale Relativbewegung zwischen Leiter 2 und Führungsrohr 5 gegenüber der Mündung 6 des Führungsrohres 5 hervortritt und durch Drehen in den Herzmuskel einschraubbar ist.

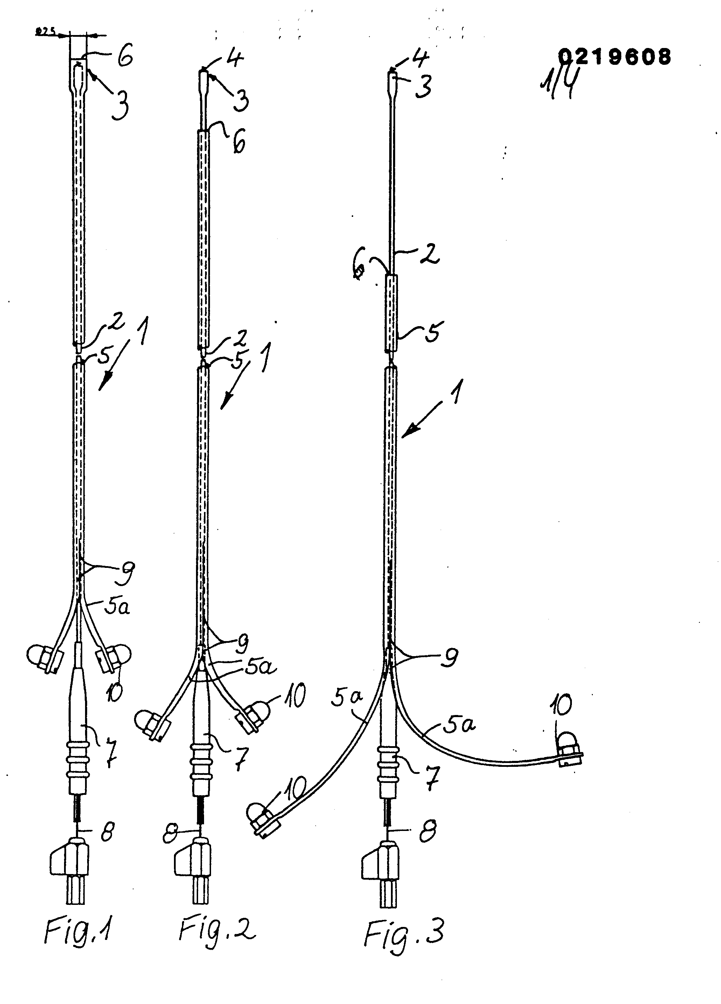

Nach dem Implantieren der Herzschrittmacherleitung 1 wird das Führungsrohr 5 in noch zu beschreibender Weise gemäß den Figuren 2 bis 4 entfernt, so daß nur noch der wesentlich dünnere Leiter 2 im Körper verbleiben muß.

Damit die Herzschrittmacherleitung 1 bzw. der Leiter 2 -

/ 7

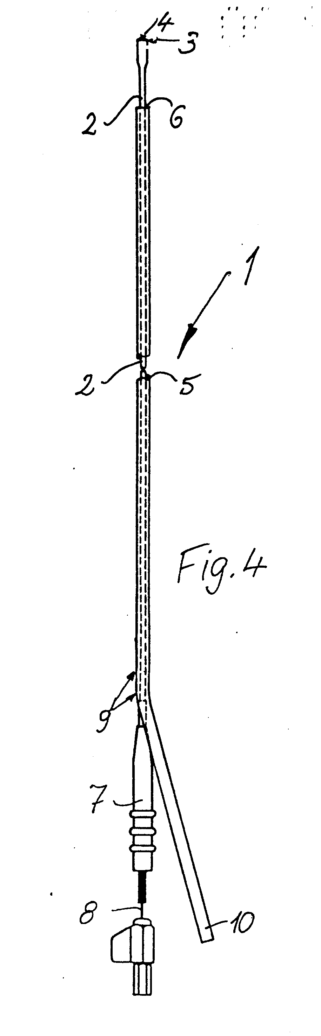

wie in den Figuren 1 bis 4 erkennbar - von vorneherein mit einem Stecker 7 versehen sein kann und die Rückzugbewegung an dem Führungsrohr 5 mittels eines Mandrin 8 im Inneren des Leiters 2 kompensiert werden kann, hat das Führungsrohr 5 gemäß Fig. 4 eine und gemäß den Fig. 1 bis 3 zwei über seine gesamte Länge verlaufende Sollreißstellen 9, die es erlauben, nach dem Implantieren der Herzschrittmacherleitung 1 das Führungsrohr 5 entgegen der Einrührrichtung nach hinten zu ziehen und außerhalb des Körpers seitlich von dem Leiter 2 ab- und vor dem Stecker 7 wegzuziehen. Das Führungsrohr 5 kann nämlich bei diesem Zurückziehen an der Sollreißlinie entsprechend dem Fortschritt des Herausziehens geöffnet werden, wie es beim Vergleich der Figuren 1 bis 3 erkennbar ist. Dadurch bleibt das proximale Ende des Leiters 2 zugänglich, so daß gemäß Fig. 1 bis 4 der Mandrin 8 eingeführt werden oder eingeführt bleiben kann und zum Gegenhalten gegen die Zugkräfte an dem Führungsrohr benutzt werden kann. Darüber hinaus stört es das Entfernen des Führungsrohres nicht, daß der Stecker 7 bereits montiert ist. Somit kann ein nachträgliches Anbringen des Steckers 7 nach dem Abziehen des Führungsrohres 5 vermieden werden.

Bei den Fig. 1 bis 3 sind am Durchmesser einander gegenüberliegend zwei Sollreißstellen über die Länge des Führungsrohres 5 vorgesehen. Somit können, wie vor allem anhand der Fig. 2 und 3 verdeutlicht, die beiden Hälften oder Teile 5 a des Führungsrohres 5 erfaßt und unter gleichzeitigem Auseinandertrennen zurückgezogen werden. Dabei kann die Sollreißstelle 9 z. B. durch eine Schwächung der Rohrwand entlang einer Meridianlinie gebildet sein.

Das Zurückziehen des Führungsrohres 5 ist dabei dadurch erleichtert, daß an dem Ende des Führungsrohres 5 an jedem Teil 5 a ein Handgriff 10 vorgesehen ist.

Es sei erwähnt, daß insbesondere bei der Ausführungsform nach Fig. 4 die Sollreißstelle 9 auch als vorgefertigter durchgehender Schlitz ausgebildet sein kann, der zunächst beim Implantieren durch die Elastizität des Werkstoffes des Führungsrohres 5 - bevorzugt Polyurethan - geschlossen ist.

Das Einführen der Herzschrittmacherleitung 1 kann mit Hilfe des Führungsrohres 5 unterstützt werden, wenn gemäß dem Ausführungsbeispiel zwischem dem Leiter 2 und dem Führungsrohr 5 in einer Wirkrichtung Formschluß besteht, der ein Zurückziehen des Leiters 2 in das Führungsrohr sperrt, das Zurückziehen des Führungsrohres 5 relativ zum Leiter 2 aber freigibt. Dies ist bei beiden Ausführungsbeispielen dadurch realisiert, daß der Querschnitt des Kopfes 3 des Leiters 2 gegenüber dem des Leiters 2 vergrößert ist und in einer entsprechenden Innendurchmesservergrößerung 11 des distalen Endes des Führungsrohres 5 angeordnet ist. Dabei kann diese Innendurchmesservergrößerung 11 des Führungsrohres durch eine Aufweitung gebildet sein, die gemäß den Fig. 2 und 3 beim Zurückziehen aufgrund der Flexibilität des Werkstoffes zurückgebildet wird. Dabei wird gleichzeitig in dieser Richtung das Zurückziehen des Führungsrohres zunächst auch ein gewisser Kraftschluß gebildet, der zwar beim Zurückziehen einfach überwunden werden kann, beim Implantieren aber ein ungewolltes vorzeitiges Austreten der Wendel 4 aus dem Führungsrohr 5 verhindern kann.

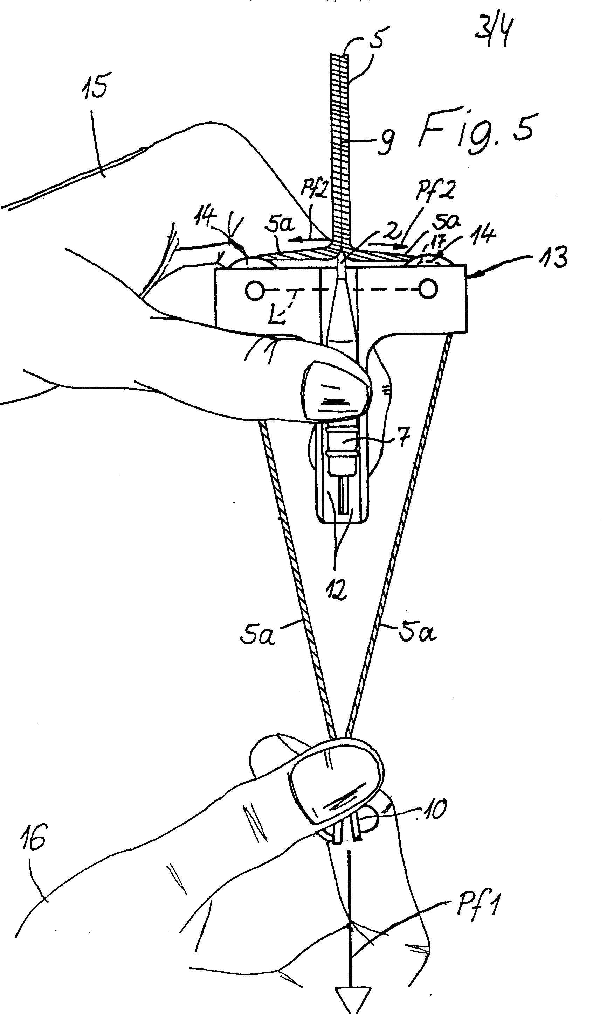

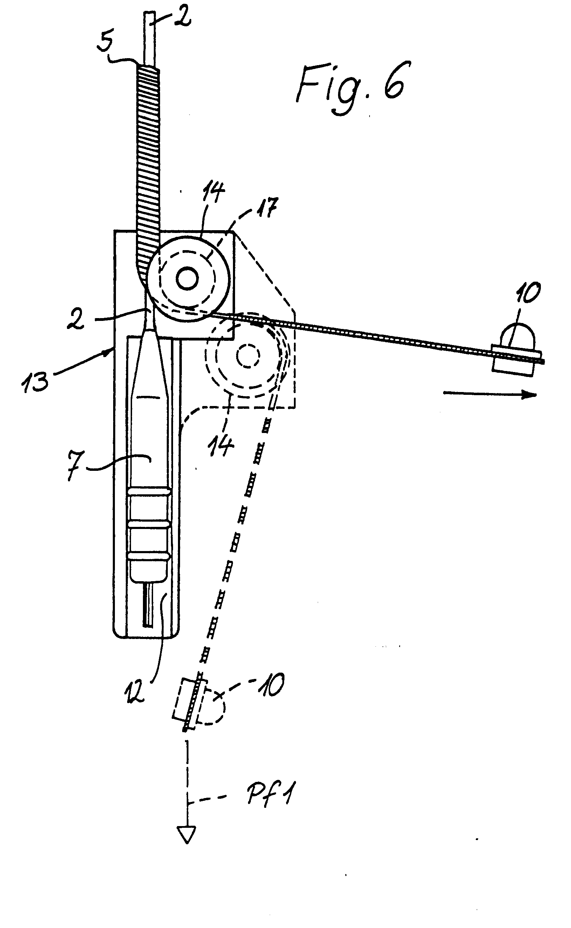

Eine besonders vorteilhafte Ausgestaltung der erfindungsgemäßen Herzschrittmacherleitungs-Anordnung vor allem für das Zurückziehen und gleichzeitige Auftrennen des Führungsrohres 5 entlang einer oder zweier Sollreißlinien 9 erkennt man in den Fig. 5 und 6. Dabei ist in beiden Fällen vorgesehen, daß der schon installierte Stecker des Leiters 2 in eine im Ausführungsbeispiel einseitig offene Haltenut 12 eines Hilfsgerätes 13 paßt, welches für das auftrennbare flexible Führungsrohr 5 seitlich der Haltenut 12 wenigstens einen Umlenkkörper 14 hat. Somit kann - wie in Fig. 5 angedeutet - der Stecker 7 mit einer Hand 15 kraftschlüssig in der Haltenut 12 gehalten werden, während das Führungsrohr 5 um einen (Fig. 6) oder zwei (Fig. 5) Umlenkkörper 14 gezogen wird, wodurch in zweckmäßiger Weise einerseits der Leiter 2 mit dem Stecker 7 festgehalten und ruhiggestellt wird, während dennoch das Führungsrohr 5 abgezogen und aufgetrennt wird, wobei die dabei wirkenden Kräfte durch das Hilfsgerät 13 von dem Leiter 2 ferngehalten werden.

Besonders vorteilhaft ist dabei die Anordnung nach Fig. 5, weil insgesamt die vom Benutzer einerseits mit der Halterhand 15 und zum anderen mit der zweiten Hand 16 aufzubringenden Kräfte einander praktisch genau entgegengesetzt sind, obwohl dennoch das Führungsrohr 5 entlang zweier Sollreißlinien 9 in zwei Führungsrohr-Teile 5 a aufgetrennt wird. Die Zugkraft der zweiten Hand 16 ist dabei durch den Pfeil Pf 1 angedeutet und entspricht einer entsprechenden Gegenhaltekraft mit Hilfe der Hand 15. Dabei erkennt man, daß die an der Sollreißstelle 9 voneinander trennbaren zwei Führungsrohr-Teile 5 a auf zwei Umlenkkörper 14 passen, die mit Abstand nebeneinander an dem Hilfsgerät 13 angeordnet sind, wobei die gedachte Verbindungslinie L der Umlenkkörper 14 quer zu dem Führungsrohr 5 und dem Leiter 2 und damit auch der Haltenut 12 und in Richtung der voneinander weggerichteten Trennkräfte gemäß den Pfeilen Pf 2 verläuft. Durch die Zugkraft gemäß dem Pfeil Pf 1 wird also das Führungsrohr nicht nur aufgespreizt und aufgerissen, sondern gleichzeitig auch zurückgezogen, wodurch sich selbsttätig auch der entsprechende Reißfortschritt ergibt.

Der Umlenkkörper 14 gemäß Fig. 6 oder die Umlenkkörper 14 gemäß Fig. 5 sind zweckmäßigerweise drehbare Rollen, die vorzugsweise mit Umfangsrillen 17 zum Aufnehmen des aufgetrennten Führungsrohres versehen sind und deren Drehachsen vorzugsweise senkrecht zu der von dem umgelenkten Führungsrohr 5 aufgespannten Ebene verlaufen.

Fig. 6 zeigt ein Hilfsgerät 13 mit einer derartigen Umlenkrolle 14 für ein Führungsrohr 5, welches nur eine Sollreißstelle hat und gegenüber dem Leiter 2 seitlich abzuziehen ist.

In all diesen Fällen ist es vorteilhaft und zweckmäßig, wenn am Ende des Führungsrohres 5 bzw. der Führungsrohrteile 5 a die Handgriffe 10 zum Aufbringen der entsprechenden Zugkräfte vorgesehen sind. Dabei verdeutlicht Fig. 5, das vor allem in Kombination mit einem Hilfsgerät 13 eine Ausführungsform eines Führungsrohres 5 mit zwei Sollreißlinien 9 einen günstigeren Kraftangriff erlaubt. Fig. 6 zeigt ein Hilfsgerät 13, mit welchem eine Anordnung gemäß Fig. 4 betätigt werden kann, bei welcher die Sollreißlinie gegebenenfalls von vorneherein als vorgefertigter durchgehender Schlitz ausgebildet ist.

In Fig. 6 ist zusätzlich mit gestrichelten Linien angedeutet, daß ein zweiter vorzugsweise als Rolle ausgebildeter Umlenkkörper 14 vorgesehen sein kann, um das schon abgezogene Führungsrohr erneut, als insgsamt etwa S- oder Z-förmig zu führen und am Handgriff 10 wiederum in Richtung des Pfeiles Pf 1 und somit entgegen der Orientierung des Leiters 2 ziehen zu können.

PATENTANWÄLTE
DIPL.-ING. H. SCHMITT
DIPL.-ING. W. MAUCHER

78 FREIBURG I. BR.
DREIKÖNIGSTR.
TELEFON: (0761) 7 07 73
7 07 74

MR/bö

0219608

11

Herr
Dr.-Ing. Peter Osypka
Basler Straße 109
7889 Grenzach-Wyhlen

UNSERE AKTE · BITTE STETS ANGEBEN!

E 86 253

Ansprüche

1. Implantierbare, transvenös einführbare Herzschrittmacherleitung (1) mit einem flexiblen, vorzugsweise gewendelten Leiter (2), welcher an seinem im Herzen zu verankernden distalen Ende eine relativ zu ihm feststehende, in den Herzmuskel einschraubbare Schraubwendel (4) aufweist, und mit einem den Leiter (2) während des Einsetzens über seine gesamte Länge umgebenden mit ihm durch die Vene zu bewegenden flexiblen Führungsrohr (5), in welchem der Leiter (2) mit seiner Isolierung während des Einführens so untergebracht ist, daß die Schraubwendel (4) nach dem Einführen der Herzschrittmacherleitung (1) in das Herz durch eine axiale Relativbewegung zwischen Leiter (2) und Führungsrohr (5) gegenüber der Mündung (6) des Führungsrohres (5) hervortritt und durch Drehen in den Herzmuskel einschraubbar ist, wobei nach dem Implantieren der Herzschrittmacherleitung (1) das Führungsrohr (5) durch die Vene zurückgezogen und aus ihr entfernt wird, d a d u r c h  g e k e n n z e i c h n e t , daß das Führungs- rohr (5) wenigstens eine über seine Länge verlaufende Sollreißstelle oder -linie (9) hat.

/ 12

2. Herzschrittmacherleitung nach Anspruch 1, dadurch gekennzeichnet, daß zwei einander vorzugsweise an einem Durchmesser gegenüberliegende Sollreißstellen (9) über die Länge des Führungsrohres (5) vorgesehen sind.

3. Herzschrittmacherleitung nach Anspruch 1 oder 2, dadurch gekennzeichnet, daß die Sollreißstelle (9) durch eine Schwächung der Rohrwand entlang einer Meridianlinie gebildet ist.

4. Herzschrittmacherleitung nach einem der Ansprüche 1 bis 3, dadurch gekennzeichnet, daß am distalen Ende der Herzschrittmacherleitung (1) zwischen Leiter (2) und Führungsrohr (5) in einer Wirkrichtung Formschluß besteht, der ein Zurückziehen des Leiters (2) in das Führungsrohr (5) sperrt und das Zurückziehen des Führungsrohres (5) relativ zum Leiter (2) freigibt.

5. Herzschrittmacherleitung nach einem der Ansprüche 1 bis 4, dadurch gekennzeichnet, daß der Querschnitt des Kopfes (3) des Leiters (2) gegenüber dem des Leiters (2) vergrößert ist und in einer entsprechenden Innendurchmesservergrößerung (11) des distalen Endes des Führungsrohres (5) angeordnet ist.

6. Herzschrittmacherleitung nach Anspruch 5, dadurch gekennzeichnet, daß die Durchmesservergrößerung (11) des flexiblen Führungsrohres (5) durch dessen Aufweitung gebildet ist.

7. Herzschrittmacherleitung nach einem der Ansprüche 1 bis 6, dadurch gekennzeichnet, daß das proximale Ende des Führungsrohres (5) wenigstens einen Handgriff (10) bei zwei Sollreißlinien (9) vorzugsweise an jedem der voneinander zu trennenden Führungsrohr-Teile (5 a) einen eigenen Handgriff (10) aufweist.

8. Herzschrittmacherleitung nach einem der vorstehenden Ansprüche, dadurch gekennzeichnet, daß die oder zumindest eine Sollreißstelle oder -linie (9) als vorgefertigter durchgehender Schlitz ausgebildet ist.

9. Herzschrittmacherleitung nach einem der vorstehenden Ansprüche, dadurch gekennzeichnet, daß am proximalen Ende des Leiters (2) ein Stecker (7) vor dem Entfernen des Führungsrohres (5) vorgefertigt befestigt ist.

10. Herzschrittmacherleitung nach einem der vorstehenden Ansprüche, dadurch gekennzeichnet, daß ihr Stecker (7) zum wenigstens kraftschlüssigen Festhalten beim Zurückziehen des Führungsrohres (5) in eine vorzugsweise einseitig offene Haltenut (12) eines Hilfsgerätes (13) paßt, welches für das auftrennbare flexible Führungsrohr (5) seitlich der Haltenut (12) und des Verlaufes des Leiters (2) wenigstens einen Umlenkkörper (14) hat.

11. Herzschrittmacherleitung nach einem der Ansprüche 1 bis 10, dadurch gekennzeichnet, daß die an der Sollreißstelle (9) voneinander trennbaren zwei Führungsrohr-Teile (5 a) auf zwei Umlenkkörper (14) passen, die mit Abstand nebeneinander an dem Hilfsgerät (13) angeordnet sind, wobei die gedachte Verbindungslinie (L) der Umlenkkörper (14) quer zu dem Führungsrohr (5) und dem Leiter (2) und in Richtung der voneinander weggerichteten Trennkräfte verläuft.

12. Herzschrittmacherleitung nach einem der vorstehenden Ansprüche, dadurch gekennzeichnet, daß der/die Umlenkkörper drehbare Rollen insbesondere mit Umfangsrollen (17) zum Aufnehmen des aufgetrennten Führungsrohres (5) bzw. der Führungsrohrteile (5 a) sind, deren Drehachsen vorzugsweise senkrecht zu der von dem umgelenkten Führungsrohr (5) aufgespannten Ebene verlaufen.

- Zusammenfassung    -

Fig.1    Fig.2    Fig.3

Fig.4

Fig. 5

Fig. 6